# EUROPEAN PATENT APPLICATION

(11) **EP 4 789 608 A1**
(43) Date of publication of application: **12.08.2026**
(21) Application number: 25156751.7
(22) Date of filing: 10.02.2025
(51) Int. Cl.: A61B 5/01, A61B 5/145, A61B 5/00, A61F 13/00

(54) **A SENSOR ASSEMBLY**

(71) Applicant: Mölnlycke Health Care AB, 431 21 Mölndal (SE)
(72) Inventor: MELIN, Daniel, 435 30 MÖLNLYCKE (SE)
(74) Representative: Kransell & Wennborg KB

(57) **Abstract**

The present disclosure generally relates to a sensor assembly (1) for use in conjunction with a medical dressing (2) or a bandage; the sensor assembly (1) comprising an electrically conductive wire (3) and a plurality of adhesive sensor strips (4a-f) arranged to extend outwardly from the wire (3), wherein each of the sensor strips (4a-f) comprises at least one sensor (5a-c) configured to monitor at least one parameter indicative of infection, wherein a distal end of the wire (3) is configured for connection with a separate processing device (6).

The present disclosure also relates to a medical dressing assembly (10) comprising the sensor assembly (1) and a medical dressing (2) or a bandage.

## Description

### TECHNICAL FIELD

The present disclosure generally relates to a sensor assembly for use in conjunction with a medical dressing or a bandage. The sensor assembly comprises an electrically conductive wire and a plurality of adhesive sensor strips. The present disclosure also relates to a medical dressing assembly comprising the sensor assembly and a medical dressing or a bandage.

### BACKGROUND

Wound infections, including surgical site infections (SSIs), represent a major challenge in post-operative and wound care management. To manage wound complications, e.g. SSIs and to secure a successful wound healing process, wound assessment is critical.

Traditionally, wound assessments have been largely qualitative, relying on visual inspections and subjective evaluations. Recent advancements have introduced the use of electrochemical sensors for quantitative wound analysis. These sensors can measure various parameters, e.g. temperature, moisture and pH, which are indicative of wound status and healing progression.

Existing sensor solutions in the field of wound care typically involve the integration of sensors and electronic circuits within wound dressings or in separate sensor sheets attached to the wound dressings. However, the integration of sensors and electronic circuits in wound dressings (or sheets) is associated with several challenges.

For example, the integration of sensors and electronic circuits in a wound dressing typically renders the dressing rigid and "bulky". This may result in discomfort for the patient, and inability of the wound dressing to conform to the contours of the skin. Accordingly, reliable wound sensing and monitoring may be impaired. Furthermore, this may result in too early detachment of the wound dressing from the skin; i.e. an impaired stay-on ability. This is particularly problematic when the wound dressing is applied onto joints or other "mobile" areas of the body.

Furthermore, precise placement of the sensors with respect to an incision (or wound) to ensure accurate readings may be a challenge. The sensors must be positioned correctly to monitor the relevant wound status parameters effectively.

Wound dressings embedded with electronic components also pose significant challenges when it comes to disposal. Such dressings are classified as hazardous waste and require careful separation of the electronic components to ensure proper disposal and recycling. This process is generally labor-intensive and costly.

Furthermore, a wound dressing embedded with sensors and other electronic components is typically complex and expensive to manufacture. Accordingly, for economical and practical reasons it is desirable that sensors and electronic components remain in contact with the skin for a prolonged time, reducing the frequency of dressing changes and ensuring continuous monitoring without interruption.

In view of this, it is desirable to provide improvements in the field of wound monitoring to secure early and reliable detection of wound infections, e.g. SSIs, while also alleviating the problems described hereinbefore.

### SUMMARY

In view of the above-mentioned problems, it is an object of the present disclosure to provide improvements in the field of wound monitoring and to provide a flexible, cost-efficient, and more sustainable means to monitor wound infections.

In a first aspect, there is provided a sensor assembly for use in conjunction with a medical dressing or a bandage; the sensor assembly comprising an electrically conductive wire and a plurality of adhesive sensor strips arranged to extend outwardly from the wire, wherein each of the sensor strips comprises at least one sensor configured to monitor at least one parameter indicative of infection, wherein a distal end of the wire is configured for connection with a separate processing device.

The present disclosure is based on the realization that the sensor assembly, when used in conjunction with a medical dressing or a bandage, significantly alleviates the problems associated with conventional dressings comprising embedded sensors and electronic components. The sensor assembly allows for a controlled application onto a surgical incision and a precise alignment of the sensors with respect to an incision (or wound).

The adhesive sensor strips, which extend outwardly from the central wire, enable a tailored and situation-adapted placement of the sensor strips directly over or around the incision (or wound) and secure accurate monitoring of the condition or status in the area of the incision (or wound). As the plurality of adhesive sensor stripes are spaced apart along the extension of the electrically conductive wire, a higher overall flexibility, i.e. lower bending rigidity (along the two-dimensional (2D) extension of the sensor assembly), can be achieved as compared with a single continuous element of the same size, e.g. a single continuous film comprising a plurality of sensors which film would have a significantly higher overall inherent bending rigidity along its 2D extension. The overall low bending rigidity, i.e. a high flexibility along the extension, of the assembly is advantageous to enable adhesive attachment of the sensor assembly to the skin and/or incision (or wound) of a patient, in particular when applied to areas of the body which are not substantial to planar.

Furthermore, the adhesive sensor strips may assist with wound or incision closure. The outward extension of the strips allows the strips to act with a suture-like mechanism and hold the incision or wound edges together. In this regard, the sensor assembly is particularly beneficial for surgical incisions (or wounds) requiring both closure and monitoring.

The sensor assembly may be used in conjunction with a medical dressing (or a bandage). Hence, the medical dressing (or bandage) may be detached from the sensor assembly (and the skin) and replaced with a new one while keeping the sensor assembly attached to the skin. For example, when a medical dressing arranged on top of the sensor assembly has become saturated or reached its full capacity, the dressing may be removed and discarded, and a new medical dressing may be arranged on top of the sensor assembly. The wear time of the sensor assembly may thus be significantly prolonged compared to conventional dressings comprising integrated sensors.

A distal end of the wire is configured for connection with a separate processing device. Accordingly, the processing device may be arranged in an area remote from the incision or wound site; e.g. in an area extending beyond a peripheral edge of a bandage or medical dressing. This allows for various maintenance activities, e.g. battery changes, data retrieval actions etc. to be carried out without disturbing the wound site or compromising the integrity of the sensor strips and the medical dressing. By having the processing device positioned away from the wound site (and the dressing or bandage), there is also less "bulk" and discomfort for the patient.

In exemplary embodiments, each of the sensor strips has a lateral and a longitudinal extension; each of the sensor strips being divided into three separate portions along the longitudinal extension: a first longitudinal edge portion, a central portion, and a second longitudinal edge portion, wherein the at least one sensor is arranged in the central portion.

Accordingly, the at least one sensor may be positioned directly above or in close vicinity of the incision (or wound) site. This way, accurate and reliable monitoring of critical wound parameters, such as pH, temperature, or moisture, may be provided in a location where they are most indicative of wound healing progress (or signs of wound infection).

In exemplary embodiments, each sensor strip may be separated from an adjacent sensor strip by a distance, d1, wherein d1 is from 15 mm to 120 mm, preferably from 20 to 80 mm.

This is beneficial when the sensor assembly is to be arranged e.g. on a knee, elbow or other joint, which moves or bends. A distance in this range allows for the central wire to be arranged with a degree of slack in critical areas such that the wire may stretch as the patient moves without straining the assembly or displacing the sensors.

It is also beneficial since it allows for adjustments and flexibility during application; i.e. it allows the medical personnel to increase or decrease the distance between the strips to accommodate various body shapes and incision (or wound) sizes and shapes.

In exemplary embodiments, the distance, d1, corresponds to or is larger than the maximum longitudinal (y) extension of each of the sensor strips.

This is beneficial for the same reasons as outlined hereinbefore. Furthermore, this arrangement prevents interference or overlap with adjacent strips during application of the strips onto the skin. The maximum longitudinal (y) extension corresponds to the length of the adhesive sensor strip.

In exemplary embodiments, each of the sensor strips has a top side and a bottom side, wherein the wire may be attached to the top side of the sensor strips, and wherein the at least one sensor is arranged on the bottom side of the sensor strips.

Accordingly, the entire bottom side of each sensor strip may be anchored to the skin (forming full skin-contact), and the wire is prevented from interfering with the adhesive contact provided between the strip(s) and the skin. This way, the stay-on ability of the sensor strips (and the sensor assembly) is improved. Furthermore, this arrangement protects the wire from direct exposure to wound exudate.

The sensors are preferably arranged in direct contact with the skin or wound surface. This facilitates accurate and reliable measurements of critical wound parameters, e.g. pH, temperature etc.

In exemplary embodiments, each of the sensor strips may comprise a polymeric film and an adhesive skin-contact layer.

The polymeric film is desirably thin and pliable such that it can stretch and conform to the movement of a wearer. The adhesive skin-contact layer secures that the sensor strips remain attached to the skin during use.

The sensors may either be attached to the adhesive skin-contact layer or they may be arranged in the adhesive skin-contact layer. For example, the at least one sensor may be arranged to protrude from the adhesive skin-contact layer.

In exemplary embodiments, the at least one sensor may be a pH sensor, a temperature sensor, a moisture sensor, and/or a sensor configured to measure dissolved molecules in a wound, preferably wherein the at least one sensor is a temperature sensor.

Each of these sensors may reveal real-time data on parameters indicative of infection, i.e the wound status and healing progression. For example, a temperature or pH sensor may detect changes associated with infection or inflammation. An infected wound typically exhibits an elevated temperature (due to increased metabolic activity and localized inflammation), and a higher pH (since the wound environment often becomes more alkaline during infection). Furthermore, a moisture sensor can assess the wound environment's hydration levels, which may be important for optimal healing

The presence and/or concentration of dissolved molecules, e.g. oxygen or ions may also provide insights into the wound status and parameters indicative of infection, e.g. oxygenation level, metabolic activity etc.

In exemplary embodiments, the at least one sensor is a first sensor, and wherein each of the sensor strips may further comprise at least one second sensor arranged in the first longitudinal edge portion or in the second longitudinal edge portion.

The second sensor may monitor conditions at the wound's (or incision's) periphery and detect early signs of spreading infection that may not yet be evident at the central wound (or incision) site.

It is also conceivable that the second sensor may be configured to measure complementary parameters, offering a more comprehensive assessment of the wound status.

Alternatively, or in addition, the second sensor may serve as a reference point or reference sensor to enhance the accuracy and reliability of the "primary" sensor (first sensor) measurements.

In exemplary embodiments, each of the sensor strips may comprise at least a first sensor arranged in the central portion, a second sensor arranged in the first longitudinal edge portion, and a third sensor arranged in the second longitudinal edge portion.

Accordingly, a more comprehensive assessment of the wound status and infection risk can be achieved. For example, the centrally arranged sensor may be arranged above or in the vicinity of the incision or wound, and the second and third sensors may be arranged remote from the incision site. The second and third sensors may be used as reference points for the first sensor, and/or may yield insight into peripheral changes, e.g. early signs of spreading infections.

According to another aspect, there is provided a medical dressing assembly comprising the sensor assembly as described hereinbefore and a medical dressing or a bandage.

In exemplary embodiments, the medical dressing or bandage is arranged on top of the sensor assembly, and wherein the distal end of the wire is arranged to extend beyond a peripheral edge of the medical dressing or the bandage during use.

As mentioned hereinbefore, this ensures that a processing device attached to the distal end of the wire remains accessible without interfering with the incision (or wound) and the medical dressing (or bandage). This arrangement facilitates battery changes, data retrieval, and other maintenance activities without disturbing the wound (or incision) site. The medical dressing and sensor assembly may still maintain consistent contact with the wound (or incision) site. Accordingly, the risk of displacement or disruption of the sensor assembly or the medical dressing (or bandage) during use is reduced, which enables consistent and reliable monitoring as well as patient comfort.

In exemplary embodiments, the medical dressing assembly comprises a medical dressing; the medical dressing comprising a backing layer and an absorbent pad arranged below the backing layer, wherein the absorbent pad is defined by pad edges; the backing layer being arranged to extend beyond the pad edges to form a border portion, wherein the medical dressing further comprises an adhesive skin-contact layer covering at least a bottom side of the border portion.

Accordingly, the area of the backing layer forming the border portion may be adhesive. The adhesive border portion surrounding the absorbent pad improves the adhesive contact with the skin, and enhances the stay-on ability, i.e. wear time of the medical dressing. Furthermore, this arrangement also facilitates detachment and replacement of the medical dressing independently of the sensor assembly.

In exemplary embodiments, each of the sensor strips of the sensor assembly is arranged below the absorbent pad during use.

Accordingly, the sensor strips are arranged underneath the absorbent pad. This way, the sensor strips are arranged in the area where the incision or wound is located (and consequently where wound exudate is present) such that the sensors can provide reliable and accurate sensor readings. Furthermore, the sensor strips are protected, i.e. shielded from external contaminants and mechanical damage.

In exemplary embodiments, the medical dressing assembly may further comprise a processing device configured for connection with the distal end of the wire, preferably wherein the processing device is arranged in a conformable pad.

The integration of the processing device within a conformable pad ensures that the processing device (and the entire medical dressing assembly) is formable, flexible, and user-friendly. The processing device can be attached to the distal end of the wire of the sensor assembly without interfering with the wound (or incision) site, or the medical dressing or bandage. This significantly simplifies the handling for medical personnel. Furthermore, the medical dressing assembly is associated with "softer" (and less bulky) components, which is convenient for the patient. All components of the medical dressing assembly can thus conform and adapt to the movement of a patient.

In exemplary embodiments, the medical dressing or bandage is void of sensors and electronic circuits.

This is beneficial as it allows the majority of the materials in the medical dressing or bandage to be handled as regular medical waste, simplifying disposal and reducing the burden on hazardous waste management. Additionally, this approach lowers the costs associated with frequent dressing changes, as the sensor assembly (comprising the more "expensive" components) can remain on the skin, while the dressing is replaced as needed.

Further features of, and advantages with, the present disclosure will become apparent when studying the appended claims and the following description. The skilled addressee realizes that different features of the present disclosure may be combined to create embodiments other than those described in the following, without departing from the scope of the present disclosure.

### BRIEF DESCRIPTION OF DRAWINGS

The various aspects of the present disclosure, including its particular features and advantages, will be readily understood from the following detailed description and the accompanying drawings, in which:
Figure 1 schematically illustrates a sensor assembly according to an exemplary embodiment of the present disclosure for positioning onto a knee of a patient.
Figure 2 schematically illustrates a medical dressing assembly according to an exemplary embodiment of the present disclosure.
Figure 3 schematically illustrates an adhesive sensor strip of the sensor assembly according to an exemplary embodiment of the present disclosure.

### DETAILED DESCRIPTION

The present disclosure will now be described more fully hereinafter with reference to the accompanying drawings, in which currently preferred embodiments of the present disclosure are shown. The present disclosure may, however, be embodied in many different forms and should not be construed as limited to the embodiments set forth herein; rather, these embodiments are provided for thoroughness and completeness, and fully convey the scope of the present disclosure to the skilled person. Like references refer to like elements throughout.

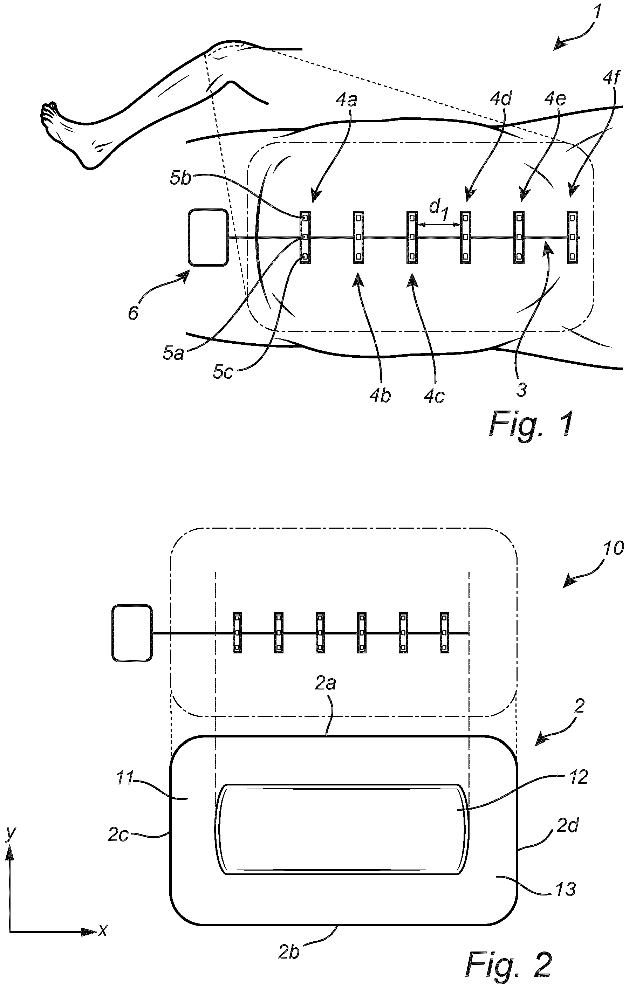

The sensor assembly will now be explained with reference to figures 1, 2 and 3.

Figure 1 illustrates a sensor assembly 1 for use in conjunction with a medical dressing 2 or a bandage; the sensor assembly 1 comprising an electrically conductive wire 3 and a plurality of adhesive sensor strips 4a-f arranged to extend outwardly from the wire 3, wherein each of the sensor strips 4a-f comprises at least one sensor 5a-c configured to monitor at least one parameter indicative of infection, wherein a distal end of the wire 3 is configured for connection with a separate processing device 6.

As used herein, the term "sensor assembly" means a system comprising an electrically conductive wire and a plurality of adhesive sensor strips connected to the wire. The sensor assembly is configured to be connected to a processing device that receives, processes, and evaluates the signals generated by the sensors. The sensor assembly is configured to operate in conjunction with a processing device and a medical dressing or a bandage.

The term "plurality of adhesive sensor strips" means at least two adhesive sensor strips, e.g. at least three adhesive sensor strips, e.g. from 3 to 8 adhesive sensor strips. The number of adhesive sensor strips may vary depending on the size of the wound (or incision), the size of the dressing, and body part to be treated.

Typically, the adhesive sensor strips are arranged at a distance from one another along the length of the wire. Consequently, the adhesive sensor strips are arranged at a distance from one another along the entire length of an incision or wound to be treated/monitored. Accordingly, sensor measurements may be accomplished along the entire incision (or wound).

The sensor strips allow for precise and controlled alignment of the sensors with respect to an incision (or wound) such that accurate sensor readings and an improved monitoring of the wound status and infection risk is accomplished.

The "sensor strip" may also be referred to as a suture sensor strip. Suture strips, also referred to as suture tapes are commonly used in a surgical setting to close an incision. Hence, the sensor strips of the present disclosure serve the dual purpose of assisting with wound or incision closure while also securing monitoring of the wound status.

Each sensor strip 4a-f has a top side and a bottom side. Typically, the at least one sensor 5a-c is arranged on the bottom side of the sensor strip.

Each sensor strip 4a-f may comprise a polymeric film and an adhesive skin-contact layer. The polymeric film forms the top side of the sensor strip. The adhesive skin-contact layer forms the bottom side of the sensor strip.

The polymeric film may comprise polyethylene, polyamide, polyester, and/or polyurethane. The thickness of the polymeric film may be in the range of from 15 to 50 µm, preferably from 20 to 40 µm.

The adhesive skin-contact layer may comprise any adhesive which is biocompatible and has a strong adhesion. Suitable materials include silicone-based adhesives, acrylic adhesives, or hydrocolloid adhesives.

The at least one sensor 5a-c may be embedded within the adhesive skin-contact layer. Alternatively, the at least one sensor 5a-c may be attached to the surface of the adhesive skin-contact layer. Preferably, the at least one sensor 5a-c is arranged to contact the skin, wound and/or incision of a patient.

The sensor(s) 5a-c may be arranged to protrude from the adhesive skin-contact layer. For example, the at least one sensor 5a-c may be arranged on the bottom side of the sensor strip, wherein the at least one sensor 5a-c is surrounded by the adhesive skin-contact layer.

The adhesive skin-contact layer ensures that the sensor strip(s) adheres firmly to the skin and provides reliable sensor readings.

The sensor(s) may be attached to the sensor strip by adhesive bonding, i.e. an adhesive may be used to secure the sensor(s) to the strip or to the adhesive skin-contact layer. Alternatively, the sensor(s) may be attached to the sensor strip(s) by mechanical attachment. For example, the sensor(s) may be arranged in a recess or groove in the adhesive skin-contact layer. It is also conceivable that the sensor(s) is encapsulated or embedded in the adhesive skin-contact layer during manufacturing.

The sensor strip is typically rectangular or substantially rectangular.

The sensor strip may have a length of from 15 mm to 100 mm, preferably from 20 mm to 60 mm. The length of the sensor strip may also be referred to as the maximum longitudinal (y) extension of the sensor strip.

The sensor strip may have a width of from 2 mm to 15 mm, preferably from 4 mm to 10 mm. The width of the sensor strip may also be referred to as the maximum lateral (y) extension of the sensor strip.

As used herein, the term "electrically conductive wire" means a flexible wire configured to transmit signals generated by one or more sensors in the adhesive sensor strips to a separate processing device. The wire comprises an electrically conductive material. For example, the wire may comprise a metal, metal alloy, e.g. copper, aluminum, silver, or gold. Alternatively, the wire may comprise a conductive polymer, or a carbon-based material, e.g. graphene.

The electrically conductive wire is preferably insulated to prevent interference or signal loss. For example, the wire may comprise an insulating coating, wherein the coating may e.g. comprise silicone, polyurethane, or polyethylene.

The length of the wire may vary depending on the size of the incision, body part to be treated, and/or the size and shape of the medical dressing or bandage to be used in conjunction with the sensor assembly.

For example, the length of the wire may be from 120 mm to 500 mm, e.g. from 200 to 400 mm.

The wire has a proximal end and a distal end. The length is measured from the proximal end to the distal end.

That the adhesive sensor strips are arranged to "extend outwardly from the wire" means that the sensor strips are connected to and positioned along the length of the wire such that they project laterally or radially away from the central axis of the wire. The adhesive sensor strips are arranged spaced apart from one another along the length of the wire.

This arrangement yields flexibility for the medical personnel when arranging the adhesive sensor strips (and sensor assembly) relative to a wound or incision. Furthermore, it allows for adaptability to wounds and incisions of various lengths and shapes while enabling precise alignment of the sensors on critical areas of interest along the wound or incision. In particular, the arrangement of the plurality of spaced apart adhesive sensor strips provides an overall higher flexibility, or low bending rigidity, of the sensor assembly in its two-dimensional extension, as compared with a single continuous layer of the same size (area of extension).

Each adhesive sensor strip 4a-f comprises at least one sensor 5a-c configured to monitor at least one parameter indicative of infection .

The at least one parameter indicative of infection is connected to the status of the wound or incision, or of the skin surrounding the wound or incision. Accordingly, information about the healing progress, infection risk or wound or skin environment may be provided such that accurate and timely detection of wound infections, e.g. SSIs can be accomplished. The sensor readings help medical personnel to quickly identify trends or anomalies. For example, a gradual increase in temperature may indicate wound infection, which may prompt timely intervention.

The at least one sensor may be configured to monitor the pH, temperature, moisture, or the concentration of dissolved molecules.

The at least one sensor may be a temperature sensor, pH sensor, moisture sensor, and/or a sensor configured to measure dissolved molecules in the wound. Preferably, the at least one sensor is a temperature sensor or a pH sensor.

Temperature sensors can detect localized increases in temperature, which may be an early indicator of infection or inflammation. The pH of the wound or skin surrounding the wound (or incision) can indicate the presence of infection or inflammation. An infected wound typically exhibits a higher, more alkaline pH compared to a healthy wound, which is slightly acidic.

A sensor configured to measure "dissolved molecules" may be a sensor configured to measure e.g. oxygen, glucose, or ions selected from hydrogen, sodium, potassium, calcium, magnesium, chloride ions, and/or ammonium ions. The presence and/or concentration of such molecules/ions may provide insights into oxygenation levels, metabolic activity, wound condition etc.

As schematically illustrated in figure 1, a distal end of the wire 3 is connected to a separate processing device 6.

As used herein, the term "processing device" means an electronic component configured to receive, process, and evaluate signals generated by the sensors in the sensor assembly. The processing device interprets the data from the sensors to provide real-time insights into the parameters monitored. The processing device may e.g. comprise a power source and means to communicate data with an electronic device.

The processing device may store, transmit, or display the processed data. For example, the processing device may be configured to communicate wirelessly or through a wired connection with an external device, such as a monitor, smartphone, or computer, for further analysis and/or remote monitoring by medical personnel.

Each of the adhesive sensor strips further comprises electrical circuits, e.g. conductive traces that connect the sensor(s) to the wire 3. The wire 3 serves as the main signal transmission pathway to the processing device 6.

The processing device 6 may be detachably attached to a distal end of the wire 3. It is also conceivable that the processing device 6 is fixedly attached to a distal end of the wire 3.

The processing device may be connected to the wire by mechanical means, e.g. clamps, clips, pluggable connectors, or snap-fit mechanisms. Alternatively, the processing device may be connected to the wire by adhesive means, or it may be integrated at e.g. the distal end of the wire.

In exemplary embodiments, the distal end of the wire 3 is attached to an adhesive electronic circuit patch. The adhesive electronic circuit patch comprises electronic circuits, which may be connected to the distal end of the wire 3. The adhesive electronic circuit patch may be arranged to extend beyond a peripheral edge of a medical dressing or bandage during use. Hence, a separate processing device may be attached to the distal end of the wire 3 via the adhesive electronic circuit patch.

As mentioned hereinbefore, the arrangement of the processing device 6 at a distal end of the wire allows it to remain accessible for data retrieval or external communication while minimizing interference with the wound or incision site or an overlying dressing or bandage.

The proximal end of the wire may be attached to an adhesive sensor strip of the sensor assembly (e.g. the sensor strip of the sensor assembly being arranged furthest away from the distal end of wire).

The sensor assembly is configured to be used in conjunction with a medical dressing or a bandage.

As used herein, the term "medical dressing" means a dressing used to treat or prevent any type of wound or injury on the skin of a patient. **In** the context of the present disclosure, wherein wound exudate is to be handled by the dressing, the medical dressing may also be referred to as a wound dressing. The wound to be treated may be any type of open or closed wound, such as a chronic wound, an acute wound, and post-operative wounds, e.g. a closed incision or scar.

As used herein, the term "bandage" means a flexible material designed to cover and protect a wound or incision, hold a dressing in place, provide support to an injured area, or apply compression. A bandage may be made from an elastic, woven, or nonwoven material. The bandage is typically configured to be wrapped around a body part. In the context of the present disclosure, the bandage may be used to secure that the sensor assembly stays in place and maintains contact with the skin or wound of a patient.

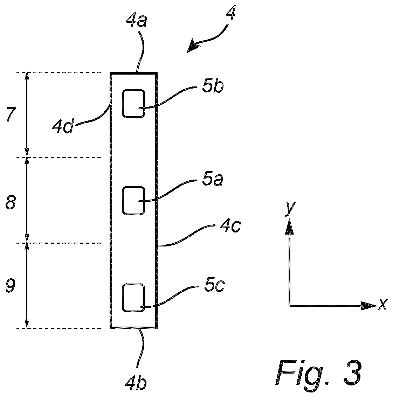

As best illustrated in figure 3, each of the sensor strips 4a-f has a lateral (x) and a longitudinal (y) extension; each of the sensor strips 4a-f being divided into three separate portions along the longitudinal (y) extension: a first longitudinal edge portion 7, a central portion 8, and a second longitudinal edge portion 9, wherein the at least one sensor may be arranged in the central portion 8.

The at least one sensor is typically arranged in an area of the sensor strip which is to be arranged above, or at least in the vicinity of, the incision (or wound).

The adhesive sensor strips may be positioned on the skin such that they cross over the incision (or wound). In other words, the at least one sensor strip is arranged perpendicularly to the incision. The first 8 and second 9 longitudinal edge portion secure that the sensor strip remains firmly adhered to the skin on both sides of the incision (or wound). By anchoring the sensor strip on both sides of the incision, the first and second longitudinal edge portions may aid in preventing displacement of the sensor strips during patient movement. Furthermore, this arrangement assists in closing an incision.

It is, of course, conceivable to arrange the sensor strips of the sensor assembly in alternative configurations. For instance, the sensor strips may be arranged in a slightly offset manner with respect to the incision, if desired.

As illustrated in figure 3, the sensor strip is defined by a first 4a and a second 4b lateral edge and a first 4c and a second 4d longitudinal edge.

A "lateral edge" of the sensor strip, the medical dressing, or the absorbent pad means an edge extending in the longitudinal (y) direction, i.e. parallel to the y axis.

A "longitudinal edge" of the sensor strip, the medical dressing, or the absorbent pad means an edge extending the lateral (x) direction, i.e. parallel to the x axis.

As best illustrated in figure 1, each sensor strip is separated from an adjacent sensor strip by a distance, d1, wherein d1 may be from 15 mm to 80 mm, preferably from 20 to 60 mm.

The distance, d1, may correspond to or may be larger than the maximum longitudinal (y) extension of each of the sensor strips 4a-b.

The distance, d1, is measured between the nearest peripheral lateral edges of two adjacent sensor strips along the length of the wire, when the wire is in a stretched condition.

A distance, d1, in the above-mentioned ranges facilitates accurate positioning of the sensor strips onto the skin and allows for flexibility and/or adjustments relative to the wound or incision during application. It also provides sufficient "spacing", i.e. separation, to prevent interference between adjacent sensor strips during application.

Each of the sensor strips 4a-f has a top side and a bottom side, wherein the wire 3 may be attached to the top side of the sensor strip 4a-f.

This is to enable full adhesive contact of the sensor strips 4a-f onto the skin of a patient.

Typically, the wire 3 is attached to the top side of the central portion 8 of the sensor strip 4.

The entire surface of the bottom side of the sensor strip is thus arranged in contact with the skin or wound of a patient. This way, an improved stay-on ability is provided, and the sensor assembly is prevented from being displaced during use.

As mentioned hereinbefore, the at least one sensor is preferably arranged on the bottom side of each sensor strip 4a-f.

The at least one sensor may be a first sensor 5a, wherein each of the sensor strips 4a-f may comprise at least one second sensor 5b; the second sensor being arranged in the first longitudinal edge portion 7 or in the second longitudinal edge portion 9.

The second sensor 5b may measure the same parameter as the first sensor 5a. For example, both sensors 5a and 5b may be configured to monitor the temperature along different areas of the strip. This may be beneficial to detect localized changes, e.g. temperature fluctuations at the edges of the incision.

Alternatively, the second sensor 5b may be configured to measure a different parameter from the first sensor 5a. For example, the first sensor 5a may be configured to measure the temperature, and the second sensor 5b may be configured to monitor the pH, moisture or concentration of dissolved molecules.

The second sensor 5b may also act as a reference sensor. This may improve the accuracy and reliability of the primary sensor (first sensor) readings.

Typically, the central portion (comprising the first sensor 5a) of the sensor strip is arranged above the wound (or incision) or in the vicinity thereof. The second sensor 5b, arranged in the first or second longitudinal edge portion, may be arranged at a distance from the incision or the wound. Accordingly, conditions at the periphery of the wound or incision may be monitored, which may indicate early signs of spreading infections.

As illustrated in figures 1-3, each of the sensor strips 4a-f may comprise at least a first sensor 5a arranged in the central portion 8, a second sensor 5b arranged in the first longitudinal edge portion 7, and a third sensor 5c arranged in the second longitudinal edge portion 9.

The provision of three sensors provides a more comprehensive assessment of the status of the wound or incision.

According to another aspect, illustrated in e.g. figure 2, there is provided a medical dressing assembly 10 comprising the sensor assembly 1 as described hereinbefore and a medical dressing 2 or a bandage.

The dotted lines in figures 1 and 2 indicate the preferred arrangement of the medical dressing 2 onto the sensor assembly 1.

The medical dressing 2 or bandage may be arranged on top of the sensor assembly 1, wherein the distal end of the wire 3 is arranged to extend beyond a peripheral edge of the medical dressing 2 or the bandage during use.

The medical dressing 2 is typically substantially rectangular (as illustrated in figure 2). However, the medical dressing is not limited to a rectangular shape, but any shape is conceivable, such as oval, square, round etc. It is also conceivable that the medical dressing has a shape adapted to fit with the sacrum or heel of a wearer.

As illustrated in figure 2, the medical dressing 2 is defined by a first 2a and a second 2b lateral edge and a first 2c and a second 2d longitudinal edge.

The dressing edges 2a-d may be straight or curved. The corners of the medical dressing may be curved.

The medical dressing 2 typically comprises a backing layer 11 and an absorbent pad 12 arranged below the backing layer 11. The backing layer 11 has a top side and a bottom side, wherein the absorbent pad is arranged to face the bottom side of the backing layer 11.

The absorbent pad 12 is defined by pad edges, wherein the backing layer 11 is arranged to extend beyond the pad edges to form a border portion 13.

The maximum width of the border portion may vary depending on the size and shape of the medical dressing. For example, the maximum width of the border portion may be from 10 to 40 mm.

The "width" of the border portion means the distance from a peripheral edge of the absorbent pad to the peripheral edge of the border portion (i.e. medical dressing dressing). This distance may vary along the peripheral edges of the medical dressing. Alternatively, it is the same around the whole periphery of the absorbent pad.

The bottom side of the backing layer may be adhesive, at least in the areas forming the border portion 13. In other words, the medical dressing may further comprise an adhesive skin-contact layer (not shown) covering at least a bottom side of the border portion 13.

It is also conceivable that the adhesive skin-contact layer also covers a bottom side of the absorbent pad 12.

In other words, the medical dressing 2 may comprise a backing layer 11, an adhesive skin-contact layer (not shown) and an absorbent pad 12 arranged between the backing layer and the adhesive skin-contact layer. The adhesive skin-contact layer may be arranged to extend beyond the pad edges to form the border portion 13.

Accordingly, the backing layer 11 and the adhesive skin-contact layer may be co-extensive in length and width.

As best illustrated in figure 2, each of the sensor strips 4a-f of the sensor assembly 1 may be arranged below the absorbent pad 12 during use (see dotted lines in figure 2).

The absorbent pad 12 is typically arranged to cover the entire incision or wound. Hence, the arrangement of the sensor strips below the absorbent pad secures that monitoring of the wound takes place in the right place, i.e. in the area of the wound of incision. Furthermore, the sensor strips are shielded by the absorbent pad such that they are protected from external contaminants, mechanical damage, and unintended displacement. Furthermore, consistent contact with the skin is provided such that reliable, uninterrupted monitoring can be accomplished.

The size of the absorbent pad may vary depending on the size and shape of the wound or incision, and the body part to be treated.

For example, the length of the absorbent pad may be from 50 to 350 mm, e.g. from 80 to 250 mm. With reference to figure 2, the length of the absorbent pad corresponds to the maximum lateral (x) extension of the absorbent pad.

The width of the absorbent pad may be from 20 to 200 mm, e.g. from 25 to 100 mm. With reference to figure 2, the width of the absorbent pad corresponds to the maximum longitudinal (y) extension of the absorbent pad

The "backing layer" is the top layer; i.e. the outermost layer of the medical dressing. The backing layer is a continuous layer and protects the layers of the dressing from entry of potential contaminants. The backing layer may comprise a polymeric film, e.g. a polyurethane film. The thickness of the backing layer may be in the range of from 15 to 50 µm, preferably from 20 to 40 µm.

The "adhesive skin-contact layer" of the medical dressing is configured to detachably adhere the dressing to a dermal surface and, in some embodiments, to the sensor assembly. The adhesive skin-contact layer may comprise a silicone-based adhesive.

The adhesive skin-contact layer may be a coating comprising a silicone-based adhesive.

The adhesive skin-contact layer may comprise a polymeric film, e.g. a polyurethane film, and a silicone-based adhesive coating; the silicone-based adhesive coating being arranged to contact the skin of a wearer during use.

The adhesive skin-contact layer may comprise a plurality of perforations in the area underlying the absorbent pad. The area of the adhesive skin-contact layer forming the border portion may be void of perforations. This may be beneficial to improve the adherence of the dressing onto the skin; i.e. to enhance the wear time of the medical dressing. It is also beneficial to prevent leakage of exudate from the absorbent pad towards the border portion.

In the context of the present disclosure, the "absorbent pad" may comprise one or a plurality of pad-forming layers. Typically, the absorbent pad comprises more than one layer.

The absorbent pad may e.g. comprise an absorbent polyurethane foam layer.

The absorbent pad may further comprise a superabsorbent layer, i.e. a layer comprising a superabsorbent material. The superabsorbent material may be superabsorbent polymer (SAP) particles or superabsorbent fibres (SAF). The superabsorbent material is capable of handling large amounts of wound exudate.

The absorbent pad may also comprise a liquid distribution layer. The liquid distribution layer may comprise any material having the ability to distribute the exudate in an efficient manner. For example, the liquid distribution layer may comprise a nonwoven material. The nonwoven may comprise viscose, polyester or blends thereof.

A layered pad construction prevents accumulation of body liquids close to the skin and improves the overall liquid handling of the medical dressing.

For example, the absorbent pad may comprise a first absorbent layer, a liquid distribution layer and a second absorbent layer. Typically, the liquid distribution layer is arranged between the first and second absorbent layers or above the first and second absorbent layers.

The liquid distribution layer distributes liquid absorbed by the first and/or second absorbent layers such that it can be evaporated through the backing layer over a large surface.

The first absorbent layer may comprise a foam, e.g. a polyurethane foam.

The second absorbent layer may be a superabsorbent layer. Accordingly, the second absorbent layer may comprise superabsorbent polymers (SAP) or superabsorbent fibers (SAF).

The layers can be joined by adhesion, lamination, using e.g. pressure and heat.

The absorbent pad may comprise additional layers, such as liquid transport layers, various combinations of foam, superabsorbent, and nonwoven layers laminated together.

A medical dressing having the construction as explained hereinabove is suitable for handling moderate to large amounts of exudate and for preventing maceration of the skin surrounding the wound. Thus, the medical dressing is particularly suited for infection prevention.

In exemplary embodiments, the processing device may be arranged in a conformable pad. The conformable pad may be formed from conventional dressing materials or be formed from injection-molded foam, plastic, or other polymeric materials. Typically, the conformable pad comprises a formable and flexible material.

It is also conceivable that the conformable pad may have the same general construction as a small wound dressing. The conformable pad may e.g. comprise a central portion and a border portion surrounding the central portion. The central portion of the pad may comprise the processing device. The border portion may be adhesive. Hence, the adhesive border portion of the pad may be attached to the skin of a patient during use.

The integration of the processing device within a conformable pad ensures that the processing device is formable, flexible, and user-friendly.

The medical dressing is preferably void of any sensors and of any electrical components.

This is beneficial from a waste management, cost efficiency, and manufacturing perspective.

The medical dressing assembly of the present disclosure may be provided as a kit of components. The kit may comprise the sensor assembly as described hereinbefore and at least one medical dressing or bandage. The kit may further comprise a separate processing device, preferably wherein the processing device is comprised in a conformable pad, as described hereinbefore. The kit may also comprise additional wound care components.

The components of the kit may be provided in the same package.

Terms, definitions and embodiments of all aspects of the present disclosure apply mutatis mutandis to the other aspects of the present disclosure.

Even though the present disclosure has been described with reference to specific exemplifying embodiments thereof, many different alterations, modifications and the like will become apparent for those skilled in the art.

Variations to the disclosed embodiments can be understood and effected by the skilled addressee in practicing the present disclosure, from a study of the drawings, the disclosure, and the appended claims. Furthermore, in the claims, the word "comprising" does not exclude other elements or steps, and the indefinite article "a" or "an" does not exclude a plurality.

## Claims

1. A sensor assembly (1) for use in conjunction with a medical dressing (2) or a bandage; said sensor assembly (1) comprising an electrically conductive wire (3) and a plurality of adhesive sensor strips (4a-f) arranged to extend outwardly from said wire (3), wherein each of said sensor strips (4a-f) comprises at least one sensor (5a-c) configured to monitor at least one parameter indicative of infection, wherein a distal end of said wire (3) is configured for connection with a separate processing device (6).

2. The sensor assembly (1) according to claim 1, wherein each of said sensor strips (4a-f) has a lateral (x) and a longitudinal (y) extension; each of said sensor strips (4a-f) being divided into three separate portions along the longitudinal (y) extension: a first longitudinal edge portion (7), a central portion (8), and a second longitudinal edge portion (9), wherein said at least one sensor (5a-c) is arranged in said central portion (8).

3. The sensor assembly (1) according to claim 1 or claim 2, wherein each sensor strip (4a-f) is separated from an adjacent sensor strip (4a-f) by a distance, d1, wherein d1 is from 15 mm to 120 mm, preferably from 20 to 80 mm.

4. The sensor assembly (1) according to claim 3, wherein the distance, d1, corresponds to or is larger than the maximum longitudinal (y) extension of each of said sensor strips (4a-b).

5. The sensor assembly (1) according to any one of the preceding claims, wherein each of said sensor strips (4a-f) has a top side and a bottom side, wherein said wire (3) is attached to said top side of said sensor strips (4a-f), and wherein said at least one sensor (5a-c) is arranged on said bottom side of said sensor strips (4a-f).

6. The sensor assembly (1) according to any one of the preceding claims, wherein each of said sensor strips (4a-f) comprises a polymeric film and an adhesive skin-contact layer.

7. The sensor assembly (1) according to any one of the preceding claims, wherein said at least one sensor (5a-c) is a pH sensor, a temperature sensor, a moisture sensor, and/or a sensor configured to measure dissolved molecules in a wound, preferably wherein said at least one sensor (5a-c) is a temperature sensor.

8. The sensor assembly (1) according to any one of claims 2-7, wherein said at least one sensor is a first sensor (5a), and wherein each of said sensor strips (4a-f) further comprises at least one second sensor (5b) arranged in said first longitudinal edge portion (7) or in said second longitudinal edge portion (9).

9. The sensor assembly (1) according to any one of claims 2-8, wherein each of said sensor strips (4a-f) comprises at least a first sensor (Sa) arranged in said central portion (8), a second sensor (5b) arranged in said first longitudinal edge portion (7), and a third sensor (5c) arranged in said second longitudinal edge portion (9).

10. A medical dressing assembly (10) comprising the sensor assembly (1) as defined in any one of claims 1-9 and a medical dressing (2) or a bandage.

11. The medical dressing assembly (10) according to claim 10, wherein said medical dressing (2) or bandage is arranged on top of said sensor assembly (1), and wherein said distal end of said wire (3) is arranged to extend beyond a peripheral edge of said medical dressing (2) or said bandage during use.

12. The medical dressing assembly (10) according to claim 10 or claim 11, wherein said medical dressing assembly (10) comprises a medical dressing (2); said medical dressing (2) comprising a backing layer (11) and an absorbent pad (12) arranged below said backing layer (11), wherein said absorbent pad (12) is defined by pad edges; said backing layer (11) being arranged to extend beyond said pad edges to form a border portion (13), wherein said medical dressing further comprises an adhesive skin-contact layer covering at least a bottom side of said border portion (13).

13. The medical dressing assembly (10) according to claim 12, wherein each of said sensor strips (4a-f) of said sensor assembly (1) is arranged below said absorbent pad (12) during use.

14. The medical dressing assembly (10) according to any one of claims 10-13, further comprising a processing device (6) configured for connection with said distal end of said wire (3), preferably wherein said processing device (6) is arranged in a conformable pad.

15. The medical dressing assembly (10) according to any one of claims 10-14, wherein said medical dressing (2) or bandage is void of sensors and electronic circuits.
